(19) Europäisches Patentamt
European Patent Office
Office européen des brevets

(11) EP 0 941 060 B1

(12) **EUROPÄISCHE PATENTSCHRIFT**

(45) Veröffentlichungstag und Bekanntmachung des Hinweises auf die Patenterteilung:
**02.04.2003 Patentblatt 2003/14**

(21) Anmeldenummer: **97948878.0**

(22) Anmeldetag: **04.11.1997**

(51) Int Cl.$^7$: **A61K 7/50**

(86) Internationale Anmeldenummer:
**PCT/EP97/06086**

(87) Internationale Veröffentlichungsnummer:
**WO 98/020840 (22.05.1998 Gazette 1998/20)**

# (54) KOSMETISCHE ZUBEREITUNGEN

COSMETIC PREPARATIONS

PREPARATIONS COSMETIQUES

(84) Benannte Vertragsstaaten:
**DE ES FR GB IT NL**

(30) Priorität: **13.11.1996 DE 19646869**

(43) Veröffentlichungstag der Anmeldung:
**15.09.1999 Patentblatt 1999/37**

(73) Patentinhaber: **Cognis Deutschland GmbH & Co. KG**
**40589 Düsseldorf (DE)**

(72) Erfinder:
- **ANSMANN, Achim**
  **D-40699 Erkrath (DE)**
- **FABRY, Bernd**
  **D-41352 Korschenbroich (DE)**

(56) Entgegenhaltungen:
**EP-A- 0 531 650**
**EP-A- 0 674 898**
**WO-A-98/03155**
**DE-A- 4 411 557**
**FR-A- 2 134 451**
**US-A- 4 919 923**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 941 060 B1

## Beschreibung

### Gebiet der Erfindung

Gegenstand der Erfindung sind kosmetische Zubereitungen, enthaltend (a) ausgewählte Perlglanzwachse, (b) Kationpolymere und (c) ausgewählte Emulgatoren sowie die Verwendung der Mischungen zur Herstellung von perlglänzenden Conditioningshampoos.

### Stand der Technik

Noch vor 15 Jahren setzten sich Haarshampoos in der Regel nur aus Wasser und Tensiden zusammen, was vom Gesichtspunkt der Reinigung und Entfettung des Haares zweifellos vollkommen ausreichend war. Mit der Aufklärung des Verbrauchers über Risiken im Umgang mit kosmetischen Inhaltsstoffen und dem Wunsch nach Produkten, die nicht nur reinigen, sondern auch pflegen, sind die Anforderungen an moderne kosmetische Zubereitungen im allgemeinen und Haarshampoos im besonderen ständig gestiegen. Der Verbraucher erwartet völlig zu Recht, daß die Produkte ein Maximum an hautkosmetischer Verträglichkeit aufweisen, d.h. daß auch bei häufigem Gebrauch durch besonders empfindliche Personen Irritationen der Haut und der Schleimhaut zuverlässig unterbleiben. Im Bereich der Pflege wird erwartet, daß die Zubereitungen auch die Kämmbarkeit der Haare verbessern, also avivierend, konditionierend und antistatisch wirken. Schließlich ist erwiesen, daß die Ästhetik der Zubereitungen, also beispielsweise ein brillanter Perlglanz, das Kaufinteresse des Verbrauchers positiv beeintlussen.

Moderne Haarshampoos des Stands der Technik enthalten aus den genannten Gründen häufig milde Tenside, Perlglanzwachse wie beispielsweise Ethylenglycolbisstearat und Kationpolymere mit avivierenden Eigenschaften. Die Formulierungsmöglichkeiten dieser Produkte sind jedoch begrenzt, da Kationpolymere in wäßrigen Lösungen nur begrenzt löslich sind, was dazu führt, daß man entweder nur geringe Mengen einsetzen kann oder Gefahr läuft, daß durch das Ausfallen der Substanzen das optische Erscheinungsbild des Produktes leidet und der Perlglanz an Brillanz verliert. Eine Übersicht zu modernen perlglänzenden Formulierungen findet sich von A.Ansmann et al. in **Parf.Kosm. 75, 578 (1994).**

Perlglanzkonzentrate, die acylierte Ethylenglycole als Perlglanzwachse zusammen mit Alkylglucosiden enthalten, sind beispielsweise aus den beiden Europäischen Patentschriften **EP-B1 0376083 und EP-B1 0570398** (Henkel) bekannt. Zusammensetzungen, die Alkyloligoglykoside und Kationpolymere beschreiben, sind aus der Patentschrift **EP-B1 0377354** (Kao) sowie aus der EP-A-0531650 bekannt.

Demzufolge hat die komplexe Aufgabe der Erfindung darin bestanden, neue kosmetische Zubereitungen, insbesondere periglänzende Conditioningshampoos, zur Verfügung zu stellen, die sich gleichzeitig durch einen brillanten Perlglanz, hohe Lagerstabilität, ausgezeichnete konditionierende und avivierende Eigenschaften und besondere hautkosmetische Verträglichkeit auszeichnen.

### Beschreibung der Erfindung

Gegenstand der Erfindung sind kosmetische Zubereitungen, enthaltend

(a) 0,1 bis 5 Gew.% Dialkylether der Formel (I),

$$R^1\text{-O-}R^2 \tag{I}$$

in der $R^1$ und $R^2$ unabhängig voneinander für lineare oder verzweigte Alkyl- und/oder Alkenylreste mit 12 bis 22 Kohlenstoffatomen stehen,

(b) 1 bis 2 Gew.% Kationpolymere und

(c) 1 bis 50 Gew.% Emulgatoren ausgewählt aus der Gruppe, die gebildet wird von Alkyl- und/oder Alkenyloligoglykosiden, Fettsäure-N-alkylpolyhydroxyalkylamiden, Alkylethersulfaten und/oder Betainen mit der Maßgabe, daß sich die Mengenangaben mit Wasser und weiteren üblichen Hilfs- und Zusatzstoffen zu 100 Gew.% ergänzen.

Überraschenderweise wurde gefunden, daß durch geeignete Auswahl von Perlglanzwachsen und Emulgatoren Mischungen erhalten werden, die in der Lage sind, Kationpolymere in wäßrigen Zubereitungen, insbesondere Haarshampoos, zu stabilisieren, so daß die Produkte die erforderliche Lagerstabilität aufweisen. Gleichzeitig stellt man beim Zusammenwirken der Distearylether und der Kationpolymeren eine synergistische Verbesserung sowohl des Perlglanzes als auch der avivierenden und konditionierenden Eigenschaften fest. Die Erfindung schließt die Erkenntnis

ein, daß die Mischungen zudem eine besonders vorteilhafte hautkosmetische Verträglichkeit aufweisen.

Dialkylether

Dialkylether, die als perlglänzende Komponente (a) in Betracht kommen, werden üblicherweise durch Kondensation entsprechender Fettalkohole herstellt **[Bull.Soc.Chim.France 333 (1949)].** Typische Beispiele sind Dilaurylether, Dimyristylether, Dicetylether, Diisostearylether, Dioleylether, Dibehenylether und Dierucylether, vorzugsweise wird Distearylether eingesetzt. Die Perlglanzwachse können eine durchschnittliche Teilchengröße im Bereich von 0,1 bis 20, vorzugsweise 5 bis 15 und insbesondere 12 bis 14 µm aufweisen.

Kationpolymere

Geeignete Kationpolymere sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z. B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, katiönischer Guar-Gum wie z.B. Jaguar® CBS, Jaguar® C-17, Jaguar® C-16 der Firma Celanese, sowie quaternierte Ammoniumsalz-Polymere wie z.B. Mirapol® A-15, Mirapol® AD-1, Mirapol® AZ-1 der Firma Miranol.

Alkyl- und/oder Alkenyloligoglykoside

Alkyl- und Alkenyloligoglykoside, die als Emulgatorkomponente (c) in Betracht kommen, stellen bekannte nichtionische Tenside dar, die der Formel (II) folgen,

$$R^3O\text{-}[G]_p \qquad (II)$$

in der $R^3$ für einen Alkyl- undloder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie, beispielsweise durch sauer katalysierte Acetalisierung von Glucose mit Fettalkoholen erhalten werden.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- undloder Alkenyloligoglykoside sind somit Alkyl- und/oder Alkenyloligo**glucoside**. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkylund/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest $R^3$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge $C_8$-$C_{10}$(DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^3$ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol,

Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

Fettsäure-N-alkylpolyhydroxyalkylamide

Fettsäure-N-alkylpolyhydroxyalkylamide, die ebenfalls als Emulgatorkomponente (c) in Betracht kommen, stellen nichtionische Tenside dar, die der Formel (III) folgen,

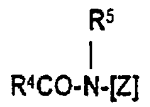

(III)

in der $R^4CO$ für einen aliphatischen Acylrest mit 6 bis 22 Kohlenstoffatomen, $R^5$ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf. Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(IV)** wiedergegeben werden:

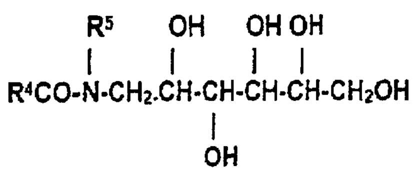

(IV)

Vorzugsweise werden als Fettsäure-N-alkylpolyhydroxyalkylamide Glucamide der Formel **(IV)** eingesetzt, in der $R^5$ für eine Alkylgruppe steht und $R^4CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (IV), die durch reduktive Aminierung von Glucose mit Methylamin und anschließende Acylierung mit Laurinsäure oder $C_{12/14}$-Kokosfettsäure bzw. einem entsprechenden Derivat erhalten werden. Weiterhin können sich die Polyhydroxyalkylamide auch von Maltose und Palatinose ableiten.

Alkylethersulfate

Neben den nichtionischen Glucosiden bzw. Glucamiden kommen als Emulgatorkomponente (c) auch anionische Tenside vom Typ der Alkylethersulfate in Frage. Alkylethersulfate ("Ethersulfate") stellen bekannte anionische Tenside dar, die großtechnisch durch $SO_3$- oder Chlorsulfonsäure (CSA)-Sulfatierung von Oxoalkohol- bzw. Fettalkoholpolyglycolethern und nachfolgende Neutralisation hergestellt werden. Im Sinne der Erfindung kommen Ethersulfate in Betracht, die der Formel **(V)** folgen,

$$R^6O\text{-}(CH_2CH_2O)_xSO_3X \qquad (V)$$

in der $R^6$ für einen linearen oder verzweigten Alkyl- und/oder Alkenylrest mit 6 bis 22 Kohlenstoffatomen, x für Zahlen von 1 bis 10 und X für ein Alkali- und/oder Erdalkalimetall, Ammonium, Alkylammonium, Alkanolammonium oder Glucammonium steht, Typische Beispiele sind die Sulfate von Anlagerungsprodukten von durchschnittlich 1 bis

10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische $C_{12/14}$- bzw. $C_{12/18}$- Kokosfettalkoholfraktionen in Form ihrer Natrium- und/oder Magnesiumsalze.

Betaine

Zur Stabilisierung der erfindungsgemäßen perlglänzenden Zubereitungen kommen als Emulgatoren, die die Komponente (c) bilden, schließlich Betaine in Betracht. Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren wie beispielsweise Acryfsäure möglich. Zur Nomenklatur und insbesondere zur Unterscheidung zwischen Betainen und "echten" Amphotensiden sei auf den Beitrag von U.Ploog in **Selfen-Öle-Fette-Wachse, 198, 373 (1982)** verwiesen. Weitere Übersichten zu diesem Thema finden sich beispielsweise von A.O'Lennick et al. in **HAPPI, Nov. 70 (1986),** S.Holzman et al. in **Tens. Det. 23, 309 (1986),** R.Bibo et al. in **Soap Cosm.Chem.Spec. Apr. 46 (1990)** und P.Ellis et al. in **Euro Cosm. 1, 14 (1994).** Beispiele für geeignete Betaine stellen die Carboxyalkylierungsprodukte von sekundären und insbesondere tertiären Aminen dar, die der Formel **(VI)** folgen,

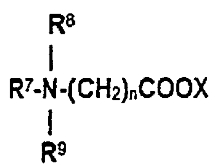

(VI)

in der $R^7$ für Alkyl- und/oder Alkenylreste mit 6 bis 22 Kohlenstoffatomen, $R^8$ für Wasserstoff oder Alkylreste mit 1 bis 4 Kohlenstoffatomen, $R^9$ für Alkylreste mit 1 bis 4 Kohlenstoffatomen, n für Zahlen von 1 bis 6 und X für ein Alkali- undloder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, $C_{12/14}$-Kokosalkyldimethylamin, Myristyldimethylamin, Cetyldimethylamin, Stearyldimethylamin, Stearylethylmethylamin, Oleyldimethylamin, $C_{16/18}$-Talgalkyldimethylamin sowie deren technische Gemische.

Weiterhin kommen auch Carboxyalkylierungsprodukte von **Amidoaminen** in Betracht, die der Formel **(VII)** folgen,

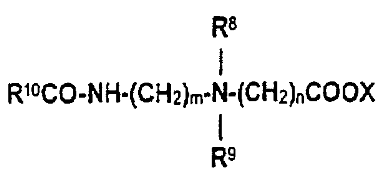

(VII)

in der $R^{10}CO$ für einen aliphatischen Acylrest mit 6 bis 22 Kohlenstoffatomen und 0 oder 1 bis 3 Doppelbindungen, m für Zahlen von 1 bis 3 steht und $R^8$, $R^9$, n und X die oben angegebenen Bedeutungen haben. Typische Beispiele sind Umsetzungsprodukte von Fettsäuren mit 6 bis 22 Kohtenstoffatomen, namentlich Capronsäure, Caprylsäure, Caprinsäure, Laurinsäure, Myristinsäure, Palmitinsäure, Palmoleinsäure, Stearinsäure, isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethyl-aminopropylamin, die mit Natriumchloracetat kondensiert werden. Bevorzugt ist der Einsatz eines Kondensationsproduktes von $C_{8/18}$-Kokosfettsäure-N,N-dimethylaminopropylamid mit Natriumchloracetat.

Weiterhin kommen als geeignete Ausgangsstoffe für die im Sinne der Erfindung einzusetzenden Betaine auch

**Imidazoline** in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum $C_{12/14}$-Kokosfettsäure, die anschließend mit Natriumchloracetat betainisiert werden.

Kosmetische Zubereitungen

Der Anteil der Komponenten (a), (b) und (c) an den erfindungsgemäßen Zubereitungen kann - bezogen auf den Feststoffgehalt - (1 bis 15) : (1 bis 15) : (70 bis 98) Gewichtsteilen unter der Voraussetzung betragen, daß sich die Mengenangaben zu 100 Gew.-% ergänzen. In der Erfindung, setzen sich die kosmetischen Zubereitungen wie folgt zusammen:

(a) 0,1 bis 5, vorzugsweise 0,5 bis 2 Gew.-% Dialkylether der Formel **(I)**,
(b) 1 bis 2 Gew.-% Kationpolymere und
(c) 1 bis 50, vorzugsweise 5 bis 25 Gew.-% Emulgatoren,
mit der Maßgabe, daß sich die Mengenangaben mit Wasser und weiteren üblichen Hilfs- und Zusatzstoffen zu 100 Gew.-% ergänzen.

**Gewerbliche Anwendbarkeit**

Die erfindungsgemäßen perlglänzenden, kationpolymerhaltigen Zubereitungen zeichnen sich durch eine hoher Lagerstabilität und brillanten Perlglanz aus. Nachdem die Kombination aus ausgewählten Perlglanzmitteln und ausgewählten Emulgatoren für die Stabilität der Zubereitungen kritisch ist, betrifft ein weiterer Gegenstand der Erfindung die Verwendung der genannten Mischungen zur Herstellung von perlglänzenden Conditioningshampoos.

Kosmetische Zubereitungen

Die erfindungsgemäßen Zubereitungen, wie beispielsweise Haarshampoos, Haarlotionen, Schaumbäder, Cremes, Lotionen oder Salben, können ferner als weitere Hilfs- und Zusatzstoffe weitere milde Tenside, Ölkörper, Co-Emulgatoren, Überfettungsmittel, Stabilisatoren, Wachse, Konsistenzgeber, Verdickungsmittel, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Typische Beispiele für geeignete milde, d.h. besonders hautverträgliche **Tenside** sind Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen, Ester von verzweigten $C_6$-$C_{13}$-Carbonsäuren mit linearen $C_6$-$C_{22}$-Fettalkoholen, Ester von linearen $C_6$-$C_{22}$-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, Ester von $C_6$-$C_{22}$-Fett-alkoholen und/oder Guerbetalkoholen mit aromatischen Carbonsäuren, insbesondere Benzoesäure, pflanzliche Öle, verzweigte primäre Alkohole, substituierte Cyclohexane, lineare $C_6$-$C_{22}$-Fettalkoholcarbonate, Ester der Benzoesäure mit $C_6$-$C_{22}$-Alkoholen, Guerbetcarbonate und/oder aliphatische bzw. naphthenische Kohlenwasserstoffe in Betracht.

Als **Co-Emulgatoren** kommen beispielsweise nichtionogene Tenside aus mindestens einer der folgenden Gruppen in Frage:

(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) $C_{12/18}$-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Anlagerungsprodukte von 15 bis 60 Mol Ethylenoxid an Ricinusöl und/oder gehärtetes Ricinusöl;
(5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(6) Anlagerungsprodukte von 2 bis 15 Mol Ethylenoxid an Ricinusöl und/oder gehärtetes Ricinusöl;
(7) Partialester auf Basis linearer, verzweigter, ungesättigter bzw. gesättigter $C_{6/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(8) Trialkylphosphate sowie Mono-, Di- und/oder Tri-PEG-alkylphosphate;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE-PS 20 24 051** als Rückfettungsmittel für kosmetische Zubereitungen bekannt. Neben den nichtionsichen kommen auch kationische Emulgatoren in Betracht, wobei solche vom Typ der Esterquats, vorzugsweise methyl-quaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacryfamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als weitere **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zink-stearat eingesetzt werden. Unter **biogenen Wirkstoffen** sind beispielsweise Tocopherol, Tocopherol-acetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind bei-spielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien

eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind

- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Die Testformulierungen wurden 14 Tage bei 20°C gelagert und anschließend bezüglich Lagerstabilität und Perlglanz subjektiv beurteilt. Es bedeuten für die Lagerstabilität : (+++) unverändert, (++) kaum merkliche Eintrübung, (+) deutliche Eintrübung und (-) Sedimentieren des Kationpolymers. Es bedeuten für den Perlglanz : (+++) brillant, (++) mäßig brillant und (+) eher stumpf. Die avivierenden Wirkung wurde über die Naßkämmbarkeit von Haarsträhnen bestimmt. Hierzu wurde die Kämmarbeit in mV für eine unbehandelte und eine gleichartige mit der Testlösung behandelte Haarsträhne gemessen. Angegeben ist die Differenz zwischen diesen beiden Wert als Mittelwert aus drei Bestimmungen. Je größer die ausgewiesene Differenz ist, um so deutlicher wird die Kämmarbeit vermindert und damit die Kämmbarkeit verbessert. Die hautkosmetische Verträglichkeit wurde über den Reizsummenscore gegenüber einer Standardrezeptur bestimmt. Die Ergebnisse sind in Tabelle 1 zusammengefaßt. Die Rezepturen 1 bis 4 sind erfindungsgemäß, die Formulierungen V1 und V2 dienen zum Vergleich.

Tabelle 1

| Zusammensetzung und Eigenschaften von perlglänzenden Shampoos mit Kationpolymeren | | | | | | |
|---|---|---|---|---|---|---|
| **INCI Bezeichnung** | **1** | **2** | **3** | **4** | **V1** | **V2** |
| Distearylether | 1,0 | 1,0 | 1,0 | 1,0 | - | 1,0 |
| Ethylenglycol Distearate | - | - | - | - | 1,0 | - |
| Lauryldimonium hydroxypropyl hydrolyzed collagen | 2,0 | - | 2,0 | 2,0 | 2,0 | 2,0 |
| Quaternized Hydroxyethyl Cellulose | - | 2,0 | - | - | - | - |
| Lauryl Glucoside | 15,0 | - | - | 5,0 | - | - |
| Sodium Laureth Sulfate | - | 12,0 | 12,0 | 10,0 | 12,0 | - |
| Cocamidopropyl Betaine | - | - | 5,0 | 2,0 | - | - |
| Laureth-2 | - | - | - | - | - | 30,0 |
| Wasser | ad 100 | | | | | |

Tabelle 1 (fortgesetzt)

| Zusammensetzung und Eigenschaften von perlglänzenden Shampoos mit Kationpolymeren | | | | | | |
|---|---|---|---|---|---|---|
| **INCI Bezeichnung** | **1** | **2** | **3** | **4** | **V1** | **V2** |
| ***Lagerstabilität*** | ++ | ++ | ++ | +++ | + | - |
| ***Perlglanz*** | +++ | +++ | +++ | +++ | ++ | + |
| ***Naßkämmbarkeit [mV]*** | 44 | 39 | 38 | 47 | 20 | 19 |
| ***Hautkosmetische Verträglichkeit [%-rel]*** | 105 | 104 | 107 | 109 | 102 | 100 |

**Patentansprüche**

1. Kosmetische Zubereitungen, **dadurch gekennzeichnet, dass** sie

   (a) 0,1 bis 5 Gew.% Dialkylether der Formel **(I)**,

$$R^1\text{-O-}R^2 \quad (I)$$

   in der $R^1$ und $R^2$ unabhängig voneinander für lineare oder verzweigte Alkyl- und/oder Alkenylreste mit 12 bis 22 Kohlenstoffatomen stehen,
   (b) 1 bis 2 Gew.% Kationpolymere und
   (c) 1 bis 50 Gew.% Emulgatoren ausgewählt aus der Gruppe, die gebildet wird von Alkylund/oder Alkenyloligoglykosiden, Fettsäure-N-alkylpolyhydroxyalkylamiden, Alkylethersulfaten und/oder Betainen.
   mit der Maßgabe enthalten, daß sich die Mengenangaben mit Wasser und weiteren üblichen Hilfsund Zusatzstoffen zu 100 Gew.-% ergänzen.

2. Zubereitungen nach Anspruch 1, **dadurch gekennzeichnet, daß** sie als Komponente (a) Distearylether enthalten.

3. Zubereitungen nach den Ansprüchen 1 und 2, **dadurch gekennzeichnet, daß** sie als Komponente (b) Kationpolymere enthalten, die ausgewählt sind aus der Gruppe, die gebildet wird von kationischen Cellulosederivaten kationischen Stärken, Copolymeren von Diallylammoniumsalzen und Acrylamiden, quatemierten Vinylpyrrolidon/ Vinylimidazol-Polymeren, Kondensationsprodukten von Polyglycolen und Aminen, quatemierten Kollagenpolypeptiden, quaternierten Weizenpolypeptiden, Polyethyleniminen, kationischen Siliconpolymeren, Copolymeren der Adipinsäure und Dimethylaminohydroxypropyldiethylentrimamin, Copolymeren der Acrylsäure mit Dimethyldiallylammoniumchlorid, Polyaminopolyamiden, kationischen Chitinderivaten, Kondensationsprodukten aus Dihalogenalkylen mit Bisdialkylaminen und/oder quaternierten Ammoniumsalz-Polymeren.

4. Zubereitungen nach den Ansprüchen 1 bis 3, **dadurch gekennzeichnet, daß** sie als Komponente (c) Alkyl- und Alkenyloligoglykoside der Formel **(II)** enthalten,

$$R^3O\text{-}[G]_p \quad (II)$$

   in der $R^3$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

5. Zubereitungen nach den Ansprüchen 1 bis 4, **dadurch gekennzeichnet, daß** sie als Komponente (c) Fettsäure-N-alkylpolyhydroxyalkylamide der Formel **(III)** enthalten.

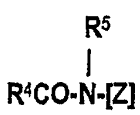

**(III)**

in der $R^4CO$ für einen aliphatischen Acylrest mit 6 bis 22 Kohlenstoffatomen, $R^5$ für einen Alkyloder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

**6.** Zubereitungen nach den Ansprüchen 1 bis 5, **dadurch gekennzeichnet, daß** sie als Komponente (c) Alkylethersulfate der Formel **(V)** enthalten,

$$R^6O\text{-}(CH_2CH_2O)_xSO_3X \qquad (V)$$

in der $R^6$ für einen linearen oder verzweigten Alkyl- und/oder Alkenylrest mit 6 bis 22 Kohfenstoffatomen, x für Zahlen von 1 bis 10 und X für ein Alkali- und/oder Erdalkalimetall, Ammonium, Alkylammonium, Alkanolammonium oder Glucammonium steht.

**7.** Zubereitungen nach den Ansprüchen 1 bis 6, **dadurch gekennzeichnet, daß** sie als Komponente (c) Betaine der Formel **(VI)** enthalten,

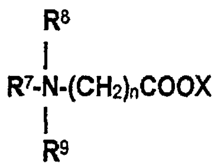

**(VI)**

in der $R^7$ für Alkyl- und/oder Alkenylreste mit 6 bis 22 Kohlenstoffatomen, $R^8$ für Wasserstoff oder Alkylreste mit 1 bis 4 Kohlenstoffatomen, $R^9$ für Alkylreste mit 1 bis 4 Kohlenstoffatomen, n für Zahlen von 1 bis 6 und X für ein Alkali- und/oder Erdalkalimetall oder Ammonium steht.

**8.** Zubereitungen nach den Ansprüchen 1 bis 7, **dadurch gekennzeichnet, daß** sie als Komponente (c) Betaine der Formel **(VII)** enthalten,

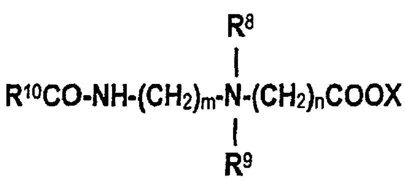

**(VII)**

in der $R^{10}CO$ für einen aliphatischen Acylrest mit 6 bis 22 Kohlenstoffatomen und 0 oder 1 bis 3 Doppelbindungen, m für Zahlen von 1 bis 3 steht und $R^8$, $R^9$, n und X die oben angegebenen Bedeutungen haben.

## Claims

**1.** Cosmetic preparations, **characterized in that** they contain

(a) 0.1 to 5% by weight of dialkyl ethers corresponding to formula **(I)**:

$$R^1\text{-}O\text{-}R^2 \qquad (I)$$

in which $R^1$ and $R^2$ independently of one another represent linear or branched alkyl and/or alkenyl groups containing 12 to 22 carbon atoms,
(b) 1 to 2% by weight of cationic polymers and
(c) 1 to 50% by weight of emulsifiers selected from the group consisting of alkyl and/or alkenyl oligoglycosides,

fatty acid-N-alkyl polyhydroxyalkylamides, alkyl ether sulfates and/or betaines,
with the proviso that the quantities shown add up to 100% by weight with water and other typical auxiliaries and additives.

**2.** Preparations as claimed in claim 1, **characterized in that** they contain distearyl ether as component (a).

**3.** Preparations as claimed in claims 1 and 2, **characterized in that** they contain as component (b) cationic polymers selected from the group consisting of cationic cellulose derivatives, cationic starches, copolymers of diallyl ammonium salts and acrylamides, quaternized vinyl pyrrolidone/vinyl imidazole polymers, condensation products of polyglycols and amines, quaternized collagen polypeptides, quaternized wheat polypeptides, polyethyleneimines, cationic silicone polymers, copolymers of adipic acid and dimethylaminohydroxypropyl diethylenetriamine, copolymers of acrylic acid with dimethyl diallyl ammonium chloride, polyaminopolyamides, cationic chitin derivatives, condensation products of dihaloalkyls with bisdialkylamines and/or quaternized ammonium salt polymers.

**4.** Preparations as claimed in claims 1 to 3, **characterized in that** they contain as component (c) alkyl and alkenyl oligoglycosides corresponding to formula **(II)**:

$$R^3O\text{-}[G]_p \qquad (II)$$

where $R^3$ is an alkyl and/or alkenyl group containing 4 to 22 carbon atoms, G is a sugar unit containing 5 or 6 carbon atoms and p is a number of 1 to 10.

**5.** Preparations as claimed in claims 1 to 4, **characterized in that** they contain as component (c) fatty acid-N-alkyl polyhydroxyalkyl amides corresponding to formula **(III)**:

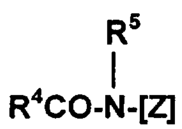

**(III)**

where $R^4CO$ is an aliphatic acyl group containing 6 to 22 carbon atoms, $R^5$ is an alkyl or hydroxyalkyl group containing 1 to 4 carbon atoms and [Z] is a linear or branched polyhydroxyalkyl group containing 3 to 12 carbon atoms and 3 to 10 hydroxyl groups.

**6.** Preparations as claimed in claims 1 to 5, **characterized in that** they contain as component (c) alkyl ether sulfates corresponding to formula **(V)**:

$$R^6O\text{-}(CH_2CH_2O)_xSO_3X \qquad (V)$$

in which $R^6$ is a linear or branched alkyl and/or alkenyl group containing 6 to 22 carbon atoms, x is a number of 1 to 10 and X is an alkali metal and/or alkaline earth metal, ammonium, alkylammonium, alkanolammonium or glucammonium.

**7.** Preparations as claimed in claims 1 to 6, **characterized in that** they contain as component (c) betaines corresponding to formula **(VI)**:

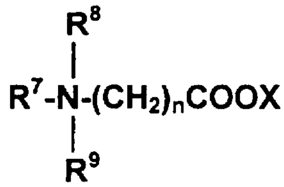

(VI)

in which $R^7$ stands for alkyl and/or alkenyl groups containing 6 to 22 carbon atoms, $R^8$ stands for hydrogen or alkyl groups containing 1 to 4 carbon atoms, $R^9$ stands for alkyl groups containing 1 to 4 carbon atoms, n is a number of 1 to 6 and X is an alkali metal and/or alkaline earth metal or ammonium.

**8.** Preparations as claimed in claims 1 to 7, **characterized in that** they contain as component (c) betaines corresponding to formula **(VII)**:

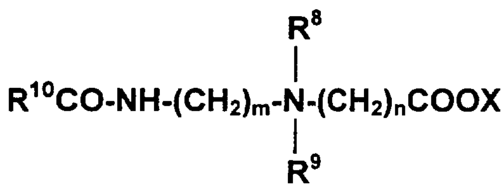

(VII)

in which $R^{10}CO$ is an aliphatic acyl group containing 6 to 22 carbon atoms and 0 or 1 to 3 double bonds, m is a number of 1 to 3 and $R^8$, $R^9$, n and X are as defined above.

## Revendications

**1.** Préparations cosmétiques
**caractérisées en ce qu'**
elles contiennent

a) de 0,1 à 5% en poids d'éthers de dialkyle de formule (I)

$$R^1\text{-O-}R^2 \quad (I)$$

dans laquelle $R^1$ et $R^2$ représentent indépendamment l'un de l'autre des radicaux alkyle et/ou alcényle linéaires ou ramifiés comportant de 12 à 22 atomes de carbone,
b) de 1 à 2% en poids de polymères cationiques et
c) de 1 à 50% en poids d'émulsifiants choisis dans le groupe formé par les alkyl- et/ou alcényloligoglycosides, les N-alkylpolyhydroxyalkylamides d'acides gras, les sulfates d'alkyléthers et/ou les bêtaïnes,
sous réserve que les indications quantitatives se complètent à 100% en poids avec de l'eau et d'autres adjuvants et additifs habituels.

**2.** Préparations selon la revendication 1,
**caractérisées en ce qu'**
elles contiennent comme composant (a) de l'éther de distéaryle.

**3.** Préparations selon les revendications 1 et 2,
**caractérisés en ce qu'**
elles contiennent comme composant (b) des polymères cationiques qui sont choisis dans le groupe formé par les dérivés cellulosiques cationiques, d'amidons cationiques, les copolymères de sels de diallylammonium et d'acrylamides, les polymères vinylpyrrolidone/vinylimidazole quatemisés, les produits de condensation de polyglycols et d'amines, les polypeptides de collagène quatemisés, les polypeptides de blé quatemisés, les polyéthylèneimines,

les polymères de silicone cationiques, les copolymères de l'acide adipique et de diméthylaminohydroxypropyldiéthylènetriamine, les copolymères de l'acide acrylique avec le chlorure de diméthyldiallylammonium, les polyaminopolyamides, les dérivés de chitine cationiques, les produits de condensation de dihalogènealkyles avec des bisdialkylamines et/ou des polymères de sels d'ammonium quaternisés.

**4.** Préparations selon les revendications 1 à 3,
**caractérisées en ce qu'**
elles contiennent comme composant (c) des alkyl- et alcényloligoglycosides de formule (II)

$$R^3O\text{-}[G]_p \qquad \text{(II)}$$

dans laquelle $R^3$ représente un radical alkyle et/ou alcényle comportant de 4 à 22 atomes de carbone, G représente un radical saccharique comportant 5 ou 6 atomes de carbone et p représente des nombres allant de 1 à 10.

**5.** Préparations selon les revendications 1 à 4,
**caractérisées en ce qu'**
elles contiennent comme composant (c) des N-alkylpolyhydroxyalkylamides d'acides gras de formule (III)

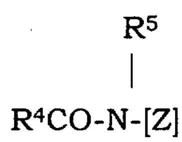

(III)

dans laquelle $R^4CO$ représente un radical acyle aliphatique comportant de 6 à 22 atomes de carbone, $R^5$ représente un radical alkyle ou hydroxyalkyle comportant de 1 à 4 atomes de carbone et [Z] représente un radical polyhydroxyalkyle linéaire ou ramifié comportant de 3 à 12 atomes de carbone et de 3 à 10 groupes hydroxyle.

**6.** Préparations selon l'une des revendications 1 à 5,
**caractérisées en ce qu'**
elles contiennent comme composant (c) des sulfates d'alkyléther de formule (V)

$$R^6O\text{-}(CH_2CH_2O)_xSO_3X \qquad \text{(V)}$$

dans laquelle $R^6$ représente un radical alkyle et/ou alcényle linéaire ou ramifié comportant de 6 à 22 atomes de carbone, x représente des nombres allant de 1 à 10 et X représente un métal alcalin et/ou alcalino-terreux, l'ammonium, un alkylammonium, alcanolammonium ou glucammonium.

**7.** Préparations selon les revendications 1 à 6,
**caractérisées en ce qu'**
elles contiennent comme composant (c) des bêtaïnes de formule (VI)

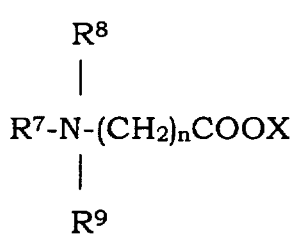

(VI)

dans laquelle $R^7$ représente des radicaux alkyle et/ou alcényle comportant de 8 à 22 atomes de carbone, $R^8$ représente un hydrogène ou des radicaux alkyle comportant de 1 à 4 atomes de carbone, $R^9$ représente un reste alkyle ayant 1 à 4 atomes de carbone, n représente des nombres allant de 1 à 6 et X représente un métal alcalin et/ou alcalino-terreux ou l'ammonium.

**8.** Préparations selon les revendications 1 à 7,
**caractérisées en ce qu'**
elles contiennent comme composant (c) des bêtaïnes de formule (VII)

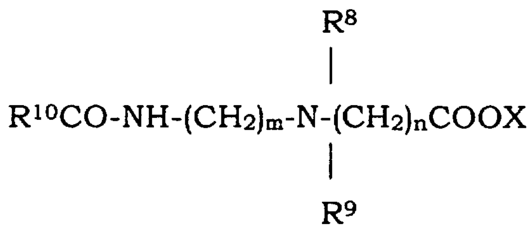

(VII)

dans laquelle $R^{10}CO$ représente un radical acyle aliphatique comportant de 6 à 22 atomes de carbone et 0 ou 1 à 3 doubles liaisons, m représente des nombres allant de 1 à 3 et $R^8$, $R^9$, n et X ont les significations indiquées ci-dessus.